# EUROPEAN PATENT APPLICATION

(11) **EP 1 090 987 A1**
(43) Date of publication of application: **11.04.2001**
(21) Application number: 99957181.3
(22) Date of filing: 23.06.1999
(51) Int. Cl.: C12N 9/12, C12N 15/54, C12N 5/10, C12Q 1/48, A61K 38/45

(54) **CELL CYCLE REGULATORY FACTOR**

(30) Priority: 23.06.1998 JP 19246798
(71) Applicant: CHUGAI SEIYAKU KABUSHIKI KAISHA, Tokyo, 115-8543 (JP)
(72) Inventor: NAKANISHI, Makoto, Nagoya-shi, Aichi 468-0023 (JP)
(74) Representative: VOSSIUS & PARTNER
(86) International application number: JP9903350
(87) International publication number: WO9967369

(57) **Abstract**

In a quest for human Cds1 (hCds1) homologous to yeast cell cycle regulating factors fission yeast spCds1 and budding yeast scCds1, GenBank EST database was searched based on spCds1 sequence and scCds1 sequence to find a *Drosophila* homologue having a significant homology to spCds1 and scCds1. Using these sequences, the full-length gene of hCds1 was isolated. The gene was strongly expressed in the testis and was widely expressed in other tissues as well. Furthermore, the protein encoded by the isolated gene was prepared as a recombinant protein, and the kinase activity thereof was detected *in vitro*, to find that the protein efficiently phosphorylated cdc25 and histone H1.

## Description

### Technical Field

The present invention relates to a protein of mammalian origin involved in the regulation of the cell cycle, and the gene thereof.

### Background Art

Cell division and proliferation comprise processes such as gene replication and mitosis, which constitute the cell cycle. The progression of the cell cycle is strictly controlled by cell cycle checkpoints. For example, when DNA is damaged, mitosis is suspended until the damage has been repaired (Science 274, 1664, 1996; Science 246, 629, 1989). Recent years have seen discoveries of cell cycle regulating factors thought to play an important role in cell cycle checkpoints (Science 277, 1450, 1997; Cell 91, 865, 1997). For example, Rad53 (also called scCds1) having a kinase activity was identified as a cell cycle regulating factor (Genes & Development 8, 2401, 1994; Genes & Development 10, 395, 1996) through a study using baker's yeast (*Saccharomyces cerevisiae*), and also, Cds1 (also called spCds1), a homologue of Rad53 (scCds1), was identified in fission yeast (*Schizosaccharomyces pombe*) (Nature 374, 817, 1995; Genes & Development 12, 382, 1998). Thus, functions of cell cycle regulating factors are being gradually revealed.

However, differing from baker's yeast and fission yeast, mammalian cells are difficult to handle, and hence, molecules corresponding to scCds1 and spCds1 have not been identified in mammals as yet. Since genes and proteins of mammalian origin could be applied for the development of pharmaceutical drugs that regulate the cell cycle, the isolation and analysis of these are extremely important.

### Disclosure of the Invention

The present invention provides a mammalian-derived gene having homology to scCds1 and spCds1, and the protein encoded by the gene. The present invention also provides a vector and a transformant cell used for producing the protein, and a method for producing a recombinant protein. The present invention provides further, an oligonucleotide utilized for the detection and isolation of the gene, an antibody used for the detection and isolation of the protein, and also, a method for screening a compound that binds to the protein and a compound that promotes or inhibits the activity of the protein.

In order to search for human Cds1 (hCds1) having homology to yeast cell cycle regulating factors scCds1 and spCds1, the inventors explored the GenBank EST database using the spCds1 sequence of fission yeast and scCds1 sequence of budding yeast, to find the homologous molecule dCds1 of fruit fly (*Drosophila*), which has a significant homology to spCds1 and scCds1. Next, the consensus sequence was extracted by aligning spCds1 gene, scCds1 gene, and the dCds1 gene found, degenerated primers were synthesized based on the sequence, and a cDNA fragment of human origin was obtained by conducting degenerated PCR using cDNA isolated from cultures of human cells as the template. Furthermore, primers were prepared based on the sequence of this cDNA fragment, and finally succeeded in isolating the full-length gene of human Cds1 (hCds1) by conducting PCR again using cDNA isolated from cultures of human cells as the template.

When northern blot analysis of human tissues was done using this gene as a probe, "hCds1" was seen to be strongly expressed in the testis, and was widely expressed in other tissues as well. When *in vitro* kinase activity was detected by preparing a "hCds1" recombinant protein, it was found that the protein efficiently phosphorylated cdc25 and histone H1. Furthermore, a mutant protein, in which the 249^{th} lysine thought to be vital for the kinase activity was converted to methionine, lost the capacity to phosphorylate these substrates.

Moreover, "hCds1" gene expression was extremely low in cell lines expressing the tumor suppressor protein p53 (normal type), and high in p53 mutant cell lines, showing that p53 expression suppressed the "hCds1" expression.

The protein of the invention is thought to regulate the cell cycle, and could be used as an important tool for the development of novel pharmaceutical drugs for the diagnosis and treatment of proliferative disorders such as cancer. Especially, compounds that inhibit the activity and expression of the protein of the present invention are anticipated to be utilized as anti-cancer agents.

The present invention relates to a novel kinase protein of mammalian origin involved in the regulation of the cell cycle, the gene thereof, molecules used in the detection, isolation, and production of the protein and gene, as well as a method for screening a compound that regulates the activity of the protein. More specifically, the present invention relates to:
(1) a protein comprising the amino acid sequence of SEQ ID NO: 1,
(2) a protein comprising the amino acid sequence of SEQ ID NO: 1, in which one or more amino acids are replaced, deleted, inserted, and/or added, and having a kinase activity,
(3) a protein which is encoded by a mammalian-derived DNA that hybridizes to the DNA comprising the nucleotide sequence of SEQ ID NO: 2, and has a kinase activity,
(4) a DNA encoding the protein of any one of (1) to (3),
(5) a vector comprising the DNA of (4),
(6) a transformant carrying the DNA of (4) in an expressible manner,
(7) a method for producing the protein of any one of (1) to (3), the method comprising a step of culturing the transformant of (6),
(8) an antibody that binds to the protein of any one of (1) to (3),
(9) a DNA specifically hybridizing to the DNA of (4) and comprising at least 15 nucleotides,
(10) a method for screening a compound having the activity to bind to the protein of any one of (1) to (3), the method comprising the steps of,
   (a) contacting a test sample with the protein of any one of (1) to (3), and,
   (b) selecting a compound having the activity to bind to the protein of any one of (1) to (3),
(11) a method for screening a compound that can promote or inhibit the kinase activity of the protein of any one of (1) to (3), the method comprising the steps of,
   (a) contacting the protein of any one of (1) to (3) with the corresponding substrate under the presence of a test compound,
   (b) assaying the substrate-targeted kinase activity of the protein of any one of (1) to (3), and
   (c) selecting a compound that can promote or inhibit the substrate-targeted kinase activity of the protein of any one of (1) to (3), by comparing the activity with that obtained in the assay under the absence of the test compound (control),
(12) a compound that can be isolated by the method of (10) or (11),
(13) the compound of (12), wherein the compound is a protein,
(14) the compound of (13), wherein the protein is an antibody,
(15) a pharmaceutical composition comprising a compound that inhibits the kinase activity of the protein of any one of (1) to (3),
(16) a pharmaceutical composition comprising a compound that inhibits the intracellular expression of the DNA of (4), and,
(17) the pharmaceutical composition of (15) or (16), wherein the pharmaceutical composition is an anti-cancer drug.

The present invention relates primarily to a mammalian-derived novel protein involved in the regulation of the cell cycle. The amino acid sequence of human-origin comprised in the protein of the invention, which was named "hCds1," is shown in SEQ ID NO: 1, and the nucleotide sequence of the cDNA encoding the protein in SEQ ID NO: 2. The "hCds1" protein isolated by the present inventors, has a significant homology to the cell cycle regulating factors scCds1 and spCds1 known in yeast, and the "hCds1" expression was observed in various human tissues, including the testis (Example 2). Though the "hCds1" protein showed a substrate-specific kinase activity *in vitro*, this kinase activity disappeared when the 249^{th} lysine was mutated, which was assumed from the amino acid sequence of yeast or *Drosophila*-derived proteins to play an important role in the kinase activity (Example 4). These facts prove that "hCds1" functions in the regulation of the cell cycle similar to the yeast and *Drosophila*-derived proteins, and that the kinase activity of the protein plays a vital role in this function. Moreover, such a relationship between the "hCds1" protein and the regulation of the cell cycle, suggests that the protein and its gene, in addition, compounds that regulate the functions of the "hCds1" protein, could be applied in the diagnosis and treatment of disorders of the cell cycle, such as cancer.

In reality, "hCds1" gene expression was low in cell lines expressing functional p53, and high in mutated p53 expressing cells (Example 5). Also, the "hCds1" gene expression was downregulated by p53 (Example 6). Thus, especially, compounds that inhibit the functions and expression of the "hCds1" protein are anticipated to be utilized as anti-cancer drugs.

Using methods commonly known to one skilled in the art, the protein of the present invention can be prepared as a natural protein or a recombinant protein utilizing genetic engineering techniques. A recombinant protein can be prepared, for example, by integrating a DNA encoding the protein of the invention (for example, a DNA comprising the nucleotide sequence of SEQ ID NO: 2) into a suitable expression vector, introducing the above vector into host cells, and purifying the protein from the transformant obtained. A natural protein may be obtained, for example, by preparing a column in which an antibody gained by immunizing small animal with the recombinant protein is immobilized, and conducting affinity chromatography using the column against an extract of tissues or cells expressing the protein of the present invention (for example, testis, etc.).

The present invention also features a protein functionally equivalent to the "hCds1" protein. In order to isolate such proteins, the method of introducing a mutation into the amino acids of the protein, is well known to those skilled in the art. As methods commonly known to those skilled in the art for modifying amino acids, those described in "Shin Saibo-Kogaku Jikken Protocol (New Cell Engineering Protocols), p241-248, Edited by the Carcinostatic Research Division, Institute of Medical Science, University of Tokyo) can be given. Mutations can also be introduced using commercially available "QuikChange Site-Directed Mutagenesis Kit" (Stratagene). The isolation of a protein functionally equivalent to the "hCds1" protein by suitably replacing an amino acid that does not influence the function of the "hCds1" protein of SEQ ID NO: 1 using the aforementioned methods, is a general procedure for one skilled in the art. Amino acid mutations can occur naturally as well. The present invention thus includes proteins functionally equivalent to the "hCds1" protein, in which one or more amino acids of the amino acid sequence shown in SEQ ID NO: 1 are replaced, deleted, inserted, and/or added. Herein, "functionally equivalent" means that the protein has a kinase activity equivalent to the natural "hCds1" protein. "Kinase activity" as used in the present invention means, the activity that produces a phosphorylated protein by transferring the phosphate group (-PO₃H₂) to a serine, threonine, or tyrosine residue of the substrate protein. The kinase activity of a protein can be detected by following the method in Example 4 described later on. The number of amino acids mutated in the protein functionally equivalent to the "hCds1" protein is not restricted, as long as a kinase activity equivalent to the "hCds1" protein is maintained. Normally, it is within 50 amino acids, preferably within 20 amino acids, more preferably, within 10 amino acids, and even more preferably within 5 amino acids. The site of mutation may be any, as long as a kinase activity equivalent to the "hCds1" protein is maintained.

The hybridization technique (for example, refer Sambrook et al., Molecular cloning 2^{nd} ed. 9.47-9.58, Cold Spring Harbor Lab. press, 1989) is well known to one skilled in the art as an alternative method for isolating a functionally equivalent protein. In other words, for one skilled in the art, it is a general procedure to prepare a protein functionally equivalent to the "hCds1" protein, by isolating DNA having a high homology to the whole or part of the DNA encoding the "hCds1" protein (SEQ ID NO: 2) on which the preparation of the protein was based. Therefore, the protein of the present invention also includes proteins that are functionally equivalent to the "hCds1" protein and are encoded by DNA hybridizing with DNA encoding the "hCds1" protein. The term "functionally equivalent" as used herein, means as mentioned above, proteins that comprise a kinase activity equivalent to the "hCds1" protein. Apart from humans, mammals such as mice, rats, dogs, rabbits, and monkeys can be used as organisms from which functionally equivalent proteins can be isolated. These proteins that are of non-human mammalian origins are useful for developing animal model systems for pharmaceutical drug development. The stringency of hybridization for isolating DNA encoding a functionally equivalent protein is, for example, 10% formamide, 5xSSPE, 1x Denhart's solution, and 1x salmon sperm DNA. More preferable (more stringent) conditions are, 25% formamide, 5xSSPE, 1x Denhart's solution, and 1x salmon sperm DNA, and even more preferable (even more stringent) conditions are, 50% formamide, 5xSSPE, 1x Denhart's solution, and 1x salmon sperm DNA. However, several factors are thought to influence the stringency of hybridization other than the above-described formamide concentration, and one skilled in the art can suitably select these factors to accomplish a similar stringency. Also, other than hybridization, it is also possible to isolate DNA encoding a functionally equivalent protein by a gene amplification method such as PCR using a portion of the DNA encoding the "hCds1" protein (SEQ ID NO: 2) as the probe.

The DNA of mammalian-origin encoding a protein that is functionally equivalent to the "hCds1" protein, and isolated by hybridization or gene amplification techniques, normally has a high homology to the DNA encoding the human-derived "hCds1" protein (SEQ ID NO: 2). "High homology" refers to a sequence identity of at least 70% or higher, preferably 80% or higher, more preferably 90% or higher, even more preferably 95% or higher. The homology of a protein can be determined by following the method in, for example, "Proc. Natl. Acad. Sci. USA 80, 726, 1983."

This invention also relates to a DNA encoding the protein of the present invention. There is no particular restriction as to the DNA of the present invention, as long as it can encode the protein of the present invention, and the DNA includes cDNA, genomic DNA, and chemically synthesized DNA. The DNA of the present invention can be isolated by using methods normally used by those skilled in the art, for example, by screening cDNA libraries or genomic libraries using the whole or part of the nucleotide sequence disclosed in SEQ ID NO: 2 as a probe, or by the PCR method using the whole or part of the nucleotide sequence as the template. The DNA of the invention can be used, for example, to produce large amounts of the protein of the invention as a recombinant protein. The recombinant protein is made by inserting DNA (for example, DNA of SEQ ID NO: 2) encoding the protein of the present invention into a suitable expression vector, introducing the vector into a suitable cell, culturing the transformants, and purifying the protein expressed.

As a host-vector system for producing the recombinant protein, numerous systems known to those skilled in the art may be used. Host cells are not especially restricted, and *E. coli*, yeast, animal cells, insect cells, and such can be given as examples. Vectors for expressing the recombinant protein within cells vary according to the host cell. For example, when using *E. coli*, pGEX (Pharmacia) and pET (Novagen), when using animal cells, pcDNA3.1 (Invitrogen), and when using insect cells, "Bac-to-Bac baculovirus expression system" (GIBCO-BRL) can be suitably used. To introduce a vector into cells, a method known to one skilled in the art, for example, electroporation, calcium phosphate method, lipofection, DEAE dextran method, and such can be used. Culturing the transformant, and separating and purifying the recombinant protein expressed by the transformant can be done by commonly used methods. When using pGEX (Pharmacia) as the vector, the expressed recombinant protein (fusion protein) can be easily purified by glutathione sepharose affinity chromatography, and when using pET (Novagen), by nickel agarose affinity chromatography.

The DNA of the invention can be applied to gene therapy. Since the DNA of the present invention is involved in the regulation of the cell cycle, the main target diseases of gene therapy are proliferative disorders like cancer. When using the DNA of the present invention for gene therapy, it is integrated into a vector to express the DNA of the invention within humans, and is introduced into the body by *in vivo* or *ex vivo* injections using methods like, the retrovirus method, liposome method, adenovirus method, etc.

The present invention also features an antibody that binds to the protein of the invention. There is no particular restriction as to the form of the antibody of the present invention and include polyclonal and monoclonal antibodies. Chimeric antibodies, humanized antibodies, and human antibodies are also included. Furthermore, not only whole antibodies, but also, Fab fragments, F(ab')₂ fragments, single-chain scFv, and such are also included. The antibody of the present invention can be prepared by methods commonly known to those skilled in the art. Polyclonal antibodies can be made by commonly known methods, for example, injecting the protein of the invention into a rabbit, and purifying the IG fraction using ammonium sulfate precipitation. Monoclonal antibodies can be made by preparing hybridoma between spleen cells of a mouse immunized with the protein of the present invention and myeloma cells, and obtaining the monoclonal antibody secreted into the culture medium, and also by transplanting the hybridoma into the peritoneal cavity to obtain the monoclonal antibody in large quantities. The antibody thus prepared, can be used for the detection and affinity purification of the protein of the invention, and can also be applied for diagnosis and antibody treatment of cell proliferative disorders like cancer caused by expression abnormalities of the protein of the invention. When using for antibody treatment, human antibodies or humanized antibodies are preferable from the viewpoint of antigenicity.

This invention also features a DNA specifically hybridizing to the DNA encoding the protein of the present invention, and comprising at least 15 nucleotides. The term "specifically hybridizing" as used herein, indicates that significant cross-hybridization does not occur with DNA encoding other proteins, under normal hybridizing conditions, preferably under, stringent hybridizing conditions. Such DNA can be utilized to detect DNA encoding the protein of the present invention, as an isolation probe, and also as a primer for amplification. Specifically, the primer in SEQ ID NO: 5 or 6 can be given as examples.

The present invention also relates to a method for screening a compound that binds to the protein of the invention. The screening method of the invention includes the steps of, (a) contacting a test sample with the protein of the invention, and (b) selecting a compound having the activity to bind to the protein of the invention. Any test sample can be used, and examples are, purified proteins (including antibodies), expression products of gene libraries, synthetic peptide libraries, cell extracts, culture supernatants, synthetic low molecular compound libraries, and such, but are not restricted thereto. Methods commonly known to one skilled in the art can be used to select a compound that has the activity to bind to the protein of the present, invention.

Using the protein of the invention, a compound that binds to it can be isolated, for example, by the following methods that are well known to one skilled in the art. A protein that binds to the protein of the invention can be prepared, for example, by creating a cDNA library from cells (for example, testis tissue cells) presumed to express a protein binding to the protein of the invention using a phage vector (λgt11, ZAP, etc.), expressing this cDNA library on LB-agarose, fixing the expressed proteins on a filter, biotin-labeling the protein of the invention or purifying it as a fusion protein with GST protein, reacting this with the above-described filter, and detecting plaques expressing the binding proteins using streptavidin or anti-GST antibody (West Western Blotting method) (Skolnik EY, Margolis B, Mohammadi M, Lowenstein E, Fischer R, Drepps A, Ullrich A, and Schlessinger J (1991) Cloning of PI3 kinase-associated p85 utilizing a novel method for expression/cloning of target proteins for receptor tyrosine kinases. Cell 65, 83-90). A protein binding to the protein of the invention can also be prepared by the "two hybrid system" ("MATCHMAKER Two-Hybrid System" "Mammalian MATCHMAKER Two-Hybrid Assay Kit," "MATCHMAKER One-Hybrid System" (Clonetech), "HybriZAP Two-Hybrid Vector System" (Stratagene), Reference-"Dalton S, and Treisman R (1992) Characterization of SAP-1, a protein recruited by serum response factor to the c-fos serum response element. Cell 68, 597-612"). In the two hybrid system, first, the protein of the invention is fused to the SRF binding region or GAL4 binding region and expressed in yeast cells, and a cDNA library that is expressed in a form that fuses with VP16 or GAL4 transcriptional activation region is prepared from cells presumed to express the protein binding to the protein of the invention, and this cDNA library is introduced into the above yeast cells. Then, cDNA derived from the library is isolated from the positive clones detected (when a protein binding to the protein of the invention is expressed in yeast cells, the binding of the two activates a reporter gene making positive clones detectable). Furthermore, the isolated cDNA is introduced into *E. coli*, and the protein encoded by the cDNA is obtained by purifying the protein expressed.

A protein binding to the protein of the invention can also be prepared by, applying the culture supernatants or cell extracts of cells predicted to express the protein binding to the protein of the invention onto an affinity column, in which the protein of the invention is immobilized, and purifying the protein that binds specifically to the column. DNA encoding the protein that binds to the protein of the invention can be obtained by: analyzing the amino acid sequence of the obtained protein, synthesizing oligo DNA based on the amino acid sequence, and screening the cDNA library using the DNA as a probe.

Othe known methods include the method of screening molecules that bind when the immobilized protein of the invention is exposed to synthetic chemical compounds, natural substance banks, or random phage peptide display libraries, and the method of screening using high-throughput based on combinatorial chemistry techniques (Wrighton NC; Farrell FX; Chang R; Kashyap AK; Barbone FP; Mulcahy LS; Johnson DL; Barrett RW; Jolliffe LK; Dower WJ., Small peptides as potent mimetics of the protein hormone erythropoietin, Science (UNITED STATES) Jul 26 1996, 273 p458-64; Verdine GL., The combinatorial chemistry of nature. Nature (ENGLAND) Nov 7 1996, 384 p11-13; Hogan JC Jr., Directed combinatorial, chemistry. Nature (ENGLAND) Nov 7 1996, 384 p17-9) to isolate low molecular weight compounds, proteins (or the genes), and peptides.

The compound thus obtained, which binds to the protein of the invention can be a candidate compound for a pharmaceutical agent for inhibiting or promoting the activity of the protein of the invention. Also, intracellular proteins that bind to the protein of the invention are thought to be closely associated with the cell cycle regulating function, specifically, the kinase activity, of the protein of the present invention. Therefore, if an intracellular protein that binds to the protein of the invention can be obtained, it would enable the development of pharmaceutical drugs aimed at disorders caused by cell cycle regulatory abnormalities, such as cancer, by screening a compound that inhibits the binding of the two.

The present invention also relates to a method for screening a compound promoting or inhibiting the activity of the protein of the invention. The screening method of the invention comprises the steps of (a) contacting the protein of the invention with the corresponding substrate under the presence of a test compound, (b) assaying the substrate-targeted kinase activity of the protein of the invention, and (c) selecting a compound that can promote or inhibit the substrate-targeted kinase activity of the protein of the invention, by comparing the activity with that obtained in the assay under the absence of the test compound (control). There are no particular restrictions as to the test compound used for the screening. Examples are, libraries of synthetic low molecular compounds, purified proteins (including antibodies), expression products of gene libraries, synthetic peptide libraries, cell extracts, culture supernatants, etc. Examples of substrates used for the detection of the kinase activity of the protein of the invention are, for example, cdc25, histone H1, or fragments thereof, but are not restricted thereto. The contact reaction of the protein of the invention and its substrate can be done, for example, as follows.

A buffer solution containing a test compound, a protein of the invention, a substrate, and ATP in which the phosphorus is radiolabeled, is prepared. After reacting the protein of the invention and the substrate for a suitable period, the kinase activity is detected. The kinase activity can be detected by the radioactivity of the phosphorus bound to the substrate. The detection of radioactivity is done by separating the solution thus reacted by electrophoresis (SDS-PAGE), drying the gel, and detecting the band of the phosphorylated substrate by autoradiography. A kinase activity is similarly assayed without the addition of the test compound for the control experiment. Next, a compound that significantly enhances or inhibits the kinase activity when compared with the control is selected. A compound thus obtained, which significantly enhances the kinase activity, becomes a candidate for a promoter of the protein of the invention, and a compound that significantly lowers the kinase activity, becomes a candidate for an inhibitor of the protein of the invention, enabling the development of pharmaceutical drugs against diseases caused by regulation defects of the cell cycle, such as cancer.

In the present invention, "hCds1" gene expression was low in various cell lines expressing functional p53 (known as a tumor suppressor protein), and on the other hand, the expression was high in p53-mutated cell lines (Example 5). Furthermore, the hCds1 gene expression was revealed to be downregulated by p53 (Example 6). These facts suggest that in normal cells, the hCds1 gene expression is suppressed by p53, and that p53 pathway is playing a major role in the DNA damage checkpoint mechanism. On the other hand, in cells malignantly transformed by p53 mutations, hCds1 expression is thought to be induced to regulate the DNA damage checkpoint mechanism in a pathway that does not mediate p53. Therefore, drugs that inhibit hCds1 activity, and drugs (drugs that inhibit the hCds1 gene expression) that specifically inhibit the hCds1 pathway in p53 mutated cancer cells, are believed to have the effect of inducing cell death in cancer cells. When calling to mind also the fact that hCds1 pathway is not functioning in normal cells, it is believed that these drugs are capable of becoming cancer-specific anti-cancer agents, without causing any side effects.

Drugs that specifically inhibit the hCds1 pathway in p53 mutated cancer cells (those that inhibit the hCds1 gene expression) can be isolated by for example, contacting candidate compounds with p53 mutated cell lines (MDAH041 cell line, HeLa cell line, H937 cell line, SaOS2 cell line, and T98B cell line), detecting hCds1 gene expression, and selecting compounds that lower the gene expression, when compared to the control where the candidate compound is not contacted. The detection of hCds1 gene expression can be done as follows: if it is in the transcription product level, by commonly known methods such as northern blotting; and if in the translation product level, by commonly known methods such as western blotting. hCds1 gene expression may also be detected by introducing a vector containing a reporter gene ligated downstream of the promoter of the hCds1 gene into a cell, and detecting the reporter activity.

The present invention encompasses a pharmaceutical composition comprising a compound that inhibits the kinase activity of the protein of the invention and a pharmaceutical composition comprising a compound that inhibits the expression of the protein of the invention, preferably a pharmaceutical composition that is an anti-cancer agent.

When using the compound obtained by the screening method of this invention as a drug for humans and mammals, for example, mice, rats, guinea pigs, rabbits, chicken, cats, dogs, sheep, pigs, cattle, monkeys, sacred baboons, and chimpanzees, the isolated compound itself can be directly administered to the patient, or it can be given after formulating by using commonly known pharmaceutical preparation methods. For example, according to the need, the drug can be taken orally as sugarcoated tablets, capsules, elixirs, and microcapsules, or parenterally in the form of injections of aseptic solutions or suspensions with water or any other pharmaceutically acceptable liquid. The compound may be formulated by mixing with, for example, pharmacologically acceptable carriers or media, specifically, sterilized water, physiological saline, plant oils, emulsifiers, suspending agents, surfactants, stabilizers, flavoring agents, excipients, vehicles, preservatives, binders, and so on, in a unit dose form required for generally accepted drug implementation. The amount of active ingredients in these preparations makes a suitable dosage within the indicated range acquirable.

Examples for additives which can be mixed to tablets and capsules are, binders such as gelatin, corn starch, tragacanth gum, and arabic gum; excipients such as crystalline cellulose; swelling agents such as corn starch, gelatin, and alginic acid; lubricators such as magnesium stearate; sweeteners such as sucrose, lactose, or saccharin; and flavoring agents such as peppermint, Gaultheria adenothrix oil, and cherry. When the unit dosage form is a capsule, a liquid carrier, such as oil, can also be included in the above ingredients. Sterile compositions for injections can be formulated following normal drug implementations using vehicles such as distilled water used for injections.

Physiological saline and isotonic liquids including glucose or other adjuvants, such as D-sorbitol, D-mannose, D-mannitol, and sodium chloride, can be used as aqueous solutions for injections. These can be used in conjunction with suitable solubilizers, such as alcohol, specifically ethanol, polyalcohols such as propylene glycol and polyethylene glycol, non-ionic surfactants, such as polysorbate 80 (TM) and HCO-50.

Sesame oil or soy-bean oil can be used as a oleaginous liquid and may be used in conjunction with benzyl benzoate or benzyl alcohol as a solubilizer; may be formulated with a buffer such as phosphate buffer and sodium acetate buffer; a pain-killer such as procaine hydrochloride; a stabilizer such as benzyl alcohol and phenol; or an anti-oxidant. The prepared injection is filled into a suitable ampule.

The administration to patients is done by methods commonly known to those skilled in the art, such as by intra-arterial, intravenous, or subcutaneous injections, and in addition, as intranasal, bronchial, intramuscular, percutaneous, or oral administrations. One skilled in the art can suitably select the dosage according to the body-weight or age of a patient, or the method of administration. Also, if the compound can be encoded by DNA, the compound can be used for gene therapy by integrating the DNA into a vector for gene therapy. Although the dosage amount and method of administration differ according to the body-weight, age, and symptoms of a patient, one skilled in the art can suitably select these.

Although there are differences according to the symptoms, the above mentioned dosage amount of compounds is, generally, about 0.1 mg to about 100 mg per day, preferably about 1.0 mg to about 50 mg, more preferably, about 1.0 mg to about 20 mg, for an adult (weight 60kg) when the compound is given orally.

When administering parenterally, although there are some differences according to the patient, target organ, symptoms, and method of administration, when giving in the form of an injection to a normal adult (body weight 60 kg), it is convenient to intravenously inject a dose of about 0.01 mg to about 30 mg per day, preferably about 0.1 mg to about 20 mg per day, and more preferably about 0.1 mg to about 10 mg per day. Also, in the case of other animals too, it is possible to administer an amount converted to 60 kg of body-weight or body-surface area.

### Brief Description of the Drawings

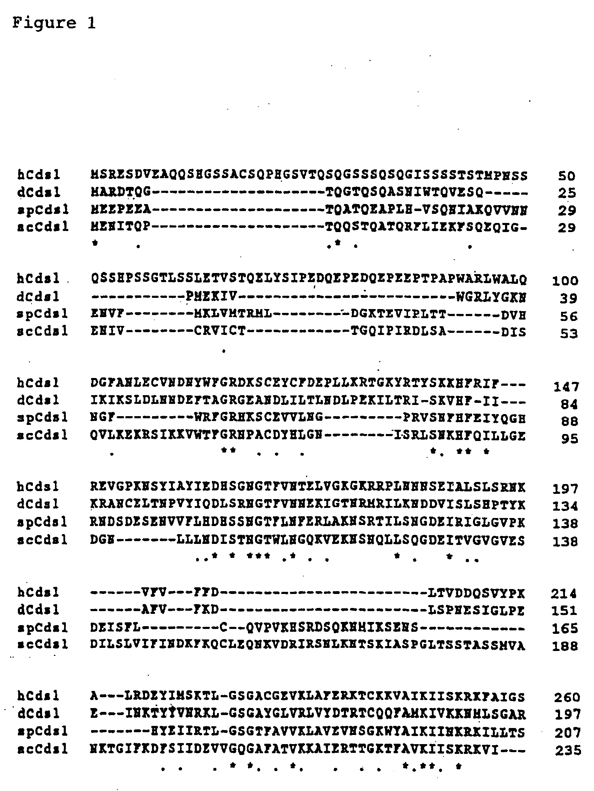

Fig. 1 shows amino acid no. 1 to no. 260 of the amino acid sequence of "hCds1" aligned with the amino acid sequences of *Drosophila* "dCds1," fission yeast "spCds1," and baker's yeast "scCds1." Amino acids are shown in one-letter codes. Hyphens show amino acid gaps. When the amino acids of the four types coincide, it is shown by an asterisk, and when similar amino acids are present, the positions are shown by dots.

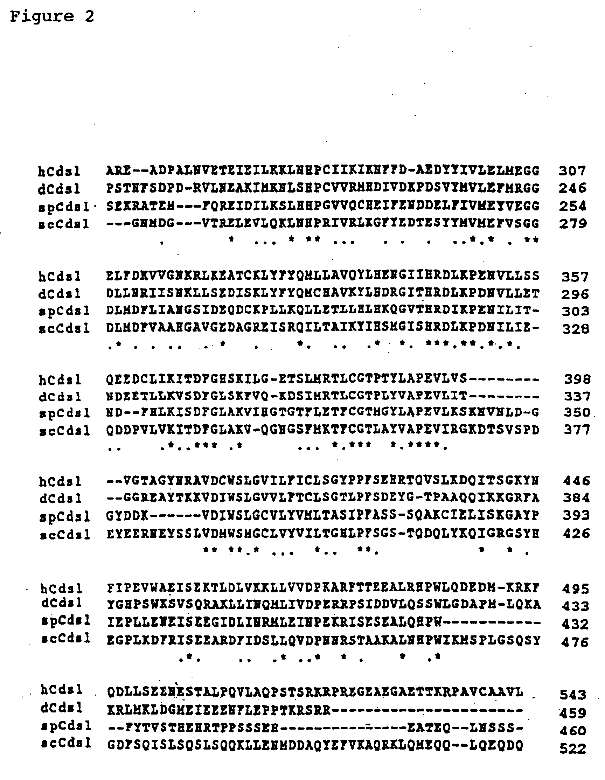

Fig. 2 shows amino acid no. 261 to no. 543 of the amino acid sequence of "hCds1" aligned with the amino acid sequences of *Drosophila* "dCds1," fission yeast "spCds1," and budding yeast "scCds1." Amino acids are shown in one-letter codes. Hyphens show amino acid gaps. When the amino acids of the four types coincide, it is shown by an asterisk, and when similar amino, acids are present, the positions are shown by dots.

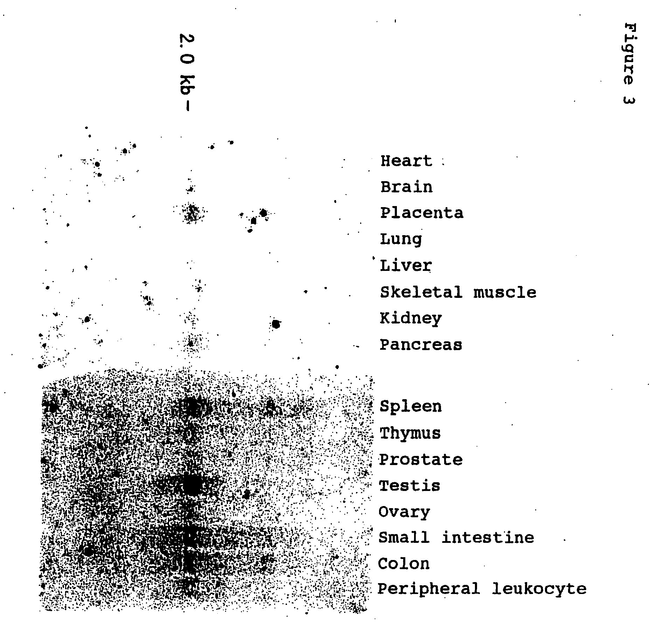

Fig. 3 shows the northern hybridization results showing "hCds1" expression in various human tissues.

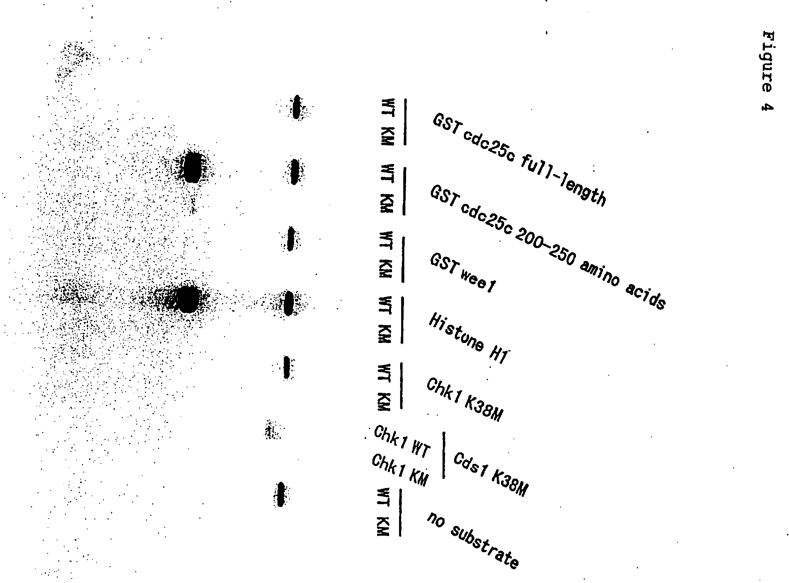

Fig. 4 shows the kinase activity of "hCds1" and "KM mutant" in which the 249^{th} lysine was converted to methionine. The "WT" below the bars of every lane shows the phosphorylation reaction by "hCds1," and "KM," the results of the phosphorylation reaction by "KM mutant." "Chk1 WT" and "Chk1 KM" indicate that the human wild-type Chk1 protein and Chk1 mutant protein in which the 38^{th} Lys was changed to Met, were used for the phosphorylation reaction, respectively. Respective substrate proteins used for the phosphorylation are shown above bars of lanes.

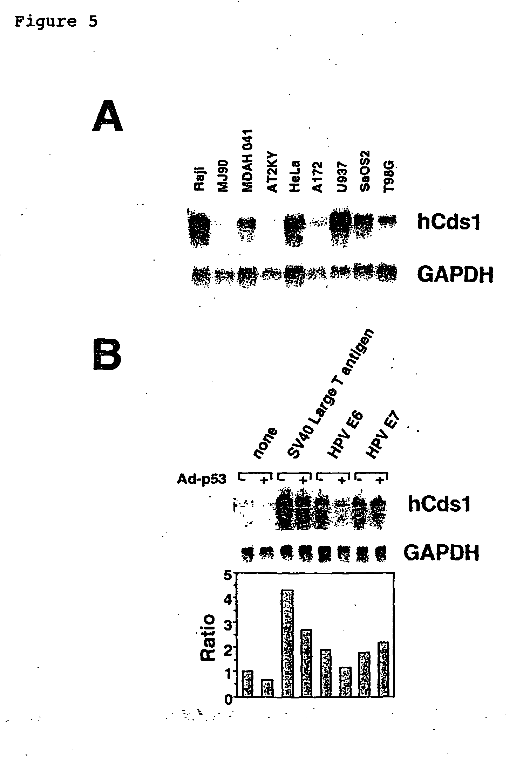

Fig. 5A shows the northern hybridization results showing hCds1 expression in various cell lines. Fig. 5B shows the northern hybridization results depicting hCds1 expression in normal fibroblasts transformed with SV40 Large T antigen, papiloma virus E6 (HPV E6), and papiloma virus E7 (HPV E7), respectively, and a graph showing the relative ratio of the positive signal.

### Best Mode for Carrying out the Invention

The present invention is described in detail with reference to examples below, but is not to be construed as being limited thereto.

### [Example 1] Identification of "hCds1" cDNA

Genbank's EST database was searched by BLAST based on fission yeast spCds1 sequence (GenBank accession no. AJ222869) and budding yeast scCds1 sequence (GenBank accession no. M55623) to newly discover the *Drosophila* homologue dCds1 (GenBank accession no. U87984), which had a 30.2% homology to spCds1, and also a homology to scCds1. Next, degenerated primers 1 and 2 (SEQ ID NO: 3 and 4) were synthesized by aligning the spCds1 gene, scCds1 gene, and dCds1 gene by using CLUSTAL V multiple sequence alignment and extracting the consensus sequence. Using these primers and cDNA prepared from MDAH041 cells as the template, degenerated PCR was done. MDAH041 cell cDNA was prepared by extracting total RNA from MDAH041 cells by the AGPC method (Analytical Biochem. 162, 156, 1987), and then, purifying mRNA by the mRNA Purification Kit (Pharmacia), and using the cDNA synthesizing kit (GIBCO-BRL) and the Gubler and Hoffman modified method (Gene, 25, 263, 1983).

The DNA fragment obtained by degenerated PCR was electrophoresed by agarose gel, purified by Gene Clean Kit II (Funakoshi), and then, inserted into the plasmid pGEM-T (Promega), and cloned. The obtained plasmid-inserted DNA had a partial sequence of 266 nucleotides, and had a homology to spCds1.

Using the partial sequence obtained and the mRNA of MDAH041 cells as the template, a cDNA fragment which was elongated in the 5*'* or 3*'* directions was prepared by 5'RACE method or 3'RACE method (GIBCO-BRL). This was amplified, and the respective nucleotide sequences determined, and "primer S-1" (SEQ ID NO: 5) and "primer α-1" (SEQ ID NO: 6) were prepared based on the obtained nucleotide sequences. The full-length "human Cds1 (hCds1)" cDNA was prepared by PCR using "primer S-1" and "primer α-1" and MDAH041 cDNA as the template. Vector pcDNA3.1/myc-HisA-hCds1 containing wild-type "hCds1" cDNA was isolated by subcloning the amplified fragment to the EcoRI/XhoI site of pcDNA3.1/myc-HiSA (Invitrogen).

The nucleotide sequence was determined using ABI PRISM Dye Terminator Cycle Sequencing Ready Reaction Kit with Amplitaq DNA polymerase FS and 377A DNA sequencer (Parkin Elmer). As a result, the full-length of app. 1.8kb "hCds1" cDNA (SEQ ID NO: 2) containing the 1629bp full-length ORF was obtained. Also, it was presumed that the protein encoded by this cDNA comprised 543 amino acids (SEQ ID NO:1). When compared with fission yeast "spCds1," budding yeast "scCds1," and *Drosophila* "dCds1," many conserved amino acids were found including the 249^{th} lysine thought to play an important role in the kinase activity. Especially, a high conservation was observed from the 220^{th} tyrosine to the 396^{th} leucine (Fig. 1 and Fig. 2).

### [Example 2] Detecting by Northern blotting

As the probe used for northern blotting, an app. 1800bp fragment obtained by treating pcDNA3.1/myc-HisA-hCds1 with EcoRI and XhoI was used. This fragment was [α-³²P]dCTP (222TBq/mmol: NewEngland Nuclear) -labeled using multiple DNA labeling systems (Amersham), and hybridization was done within an aqueous solution containing 5xSSPE, 5xDenhardt's, 50% formamide, 0.5% SDS, and 100 µg/ml heat-denatured salmon sperm DNA at 42°C for 24 hrs against the Multiple Tissue Northern Blots (Clontech) filters. As a result, a strong expression was seen in the testis and was widely expressed in other tissues as well (Fig. 3).

### [Example 3] Preparation of "hCds1" protein and mutant protein

Homologous recombination into Baculovirus DNA was done using Sf9 cells as insect cells (Pharmingen), and TNM-FH (Pharmingen) as medium, and the transfection was done using Baculo Gold Transfection Kit (Pharmingen). After excising the cDNA fragment from "hCds1" cDNA inserted vector pcDNA3.1/myc-HisA-hCds1 using restriction enzymes EcoRI and PmeI, it was inserted into the EcoRI/SmaI restriction enzyme site of transfer vector pVL1392 (pVL1392 hCds1-myc/6xHis). Sf9 cells (1x10⁶ cells) were plated on a 35mm dish, and allowed to stand for one hour at room temperature to attach the cells onto the plate. After washing the cells with a serum-free culture medium for insect cells (Sf-900 II SFM, Gibco-BRL), 0.5 ml of transfection solution A was added. The co-transfection solution was prepared by mixing 500 ng of pVL1392 hCds1-myc/6xHis, 2.5 ng of linear Baculo Gold Virus DNA (Pharmingen), 0.05 ml of sterilized water, and 0.5 ml of transfection solution B, and leaving this mixture at room temperature for 15 min. This co-transfection mixture (0.1 ml) was slowly dropped into the dish containing 0.5 ml of transfection solution A and Sf9 cells, and after leaving at 27°C for 4 hrs, the dish was washed, 2 ml of TNM-FH medium was added, and the culture supernatant was collected after culturing at 27°C for 5 days to prepare the virus stock.

The homologous recombination into baculovirus DNA was verified by a plaque assay. Sf9 cells (1x10⁶ cells) were plated on a 35 mm dish, and after adding the virus stock diluted 10⁻¹ to 10⁻³, the dish was shaken for one hour at room temperature. After removing the virus stock, 3 ml of a mixture of 2.5% baculovirus agarose and TNM-FH medium mixed at 1:3 (42°C) was poured in, and after the agarose hardened, the dish was cultured at 27°C for 5 days. The resulting plaques were picked up with a Pasteur pipette, and after suspending in 0.5 ml of medium, were left aside overnight at 4°C, to prepare a plaque pick solution.

After plating 1x10⁶ Sf9 cells on a 35 mm dish to attach onto, 0.1 ml of plaque pick solution was added, and the dish was shaken gently at room temperature for one hour. TNM-FH medium (1.5 ml) was added thereto, and further cultured at 27°C for 5 days. Cells and culture supernatant were collected, and the culture supernatant obtained by centrifuging at 3000rpm for 5 minutes, was used as the P-1 solution. The cells obtained by centrifugation were treated with protease K and RNaseA, and after removing proteins by a further treatment with phenol/chloroform, the presence or absence of the target inserts was verified by PCR. P-1 solutions having the objective inserts were preserved, and titer-check was conducted for these P-1 solutions.

The recombinant "hCds1" was expressed in insect cells as follows. Sf9 cells (1x10⁶ cells) were plated on a 35 mm dish, and the cells were infected with P-1 solution at M.O.I.=30. After culturing at 27°C for 3 days, cells expressing recombinant "hCds1" were collected. Cells were lysed in a cell lysis buffer (50 mM HEPES pH8.0, 150 mM NaCl, 1 mM EDTA, 2.5 mM EGTA, 10% glycerol, 0.1% NP-40, 2 mg/ml aprotinin, 0.1 mM PMSF, 1 mM NaF, 0.1 mM Na₃VO₄, 10 mM β-glyceropbosphate) to prepare a cell lysed solution. This solution was mixed with anti-myc antibody-binding protein A beads to bind the objective recombinant "hCds1" to beads. After washing the beads with cell lysis buffer, recombinant "hCds1" was obtained as an immunoprecipitate.

In order to determine the site involved in kinase activation, the gene encoding the mutant (KM mutant), in which the 249^{th} lysine was converted to methionine, was prepared by PCR. After conducting PCR using wild type "hCds1" cDNA as the template and each primer set of "primer S-1" and "inner primer 2" (SEQ ID NO: 7), or "primer α-1" and "inner primer 1" (SEQ ID NO: 8), the both of amplified fragments were mixed and PCR was done again by primer S-1 and primer α-1, and the amplified fragment was subcloned into the EcoRI/XhoI site of pcDNA3.1/myc-HisA. The mutant protein was expressed in insect cells by the same method used for expressing the wild type protein.

### [Example 4] Detection of kinase activity

The immunoprecipitants of wild-type and mutant "hCds1" were suspended in kinase buffer (10 mM MgCl₂, 50 mM HEPES pH 8.0, 2.5 mM EGTA, 1 mM DTT, 0.1 mM PMSF, 2 mg/ml aprotinin, 10 mM β-glycerophosphate, 0.1 mM Na₃VO₄, 1 mM NaF, 10 mM ATP, 185, kBq [γ-³²P]ATP(222TBq/mmol (NewEngland Nuclear)). "GST-cdc25c (full-length) (Accession number L26584)," "GST-cdc25c (200 to 250 amino acids)," "GST-weel (Accession number U10564)," histone H1 (J. Biochem. 100, 359, 1986), and human Chk1 (Accession number AF016582)proteins prepared as a fusion protein with glutathione S-transferase (GST) were added as substrates, and incubated at 30°C for 30 min. The reaction solution was separated by SDS-PAGE (12%), and after the gel was dried, the band of phosphorylated protein was detected by autoradiography. As a result, it was found that recombinant "Cds1" efficiently phosphorylated "GST-cdc25c (full length)," "GST-cdc25 (200 to 250 amino acids)," and histone H1 but hardly phosphorylated "GST-weel," and human Chk1 protein (Fig. 4). The mutant (KM mutant) in which the 249^{th} lysine was converted to methionine, failed to phosphorylate any substrate. The fusion protein with glutathione-S-transferase (GST) was prepared as described in reference Genes & Development 12, 382, 1998.

### [Example 5] Correlation between p53 mutation and hCds1 expression

mRNA was purified from each type of cultured cells, using Pharmacia Quick prep. mRNA kit, 2 µg of each was transferred to a nylon membrane following agarose gel electrophoresis, and northern blotting was done using the hCds1 fragment as the probe. Detection done using hGAPDH for the probe was used as the control.

As a result, hCds1 expression was extremely low in MJ90, AT2KY, and A172 cell lines expressing functional p53, but was high in p53 mutant cell lines MDAH041, HeLa, U937, SaOS2, and T98G (Fig. 5A). This fact revealed that hCds1 expression correlates extremely well with the absence or presence of a mutation in the p53 gene. Also, the regulation of hCds1 expression was suggested to occur via p53.

### [Example 6] Influence of p53 forced-expression on hCds1 expression

Normal fibroblast cells were transformed with SV40 Large T, papiloma virus E6, and E7, respectively, and the change in hCds1 expression was analyzed by northern blotting method using the same method described above. Also, the change in hCds1 expression was examined when p53 was exogenously expressed by infecting each cell with wild-type p53-containing adenovirus.

As a result, when normal fibroblast cells were transfected with SV40 Large T, papiloma virus E6, and E7, a strong hCds1 expression was induced. Although hCds1 expression was suppressed by normal p53 expression in cells transfected with Large T and E6, no influence was seen in cells transfected with E7 (Fig. 5B). This fact coincides with the report that although Large T and E6 transfect cells by inhibiting p53 function, E7 suppresses Rb, and does not influence p53. Namely, it was suggested that the hCds1 expression was downregulated by p53.

### Industrial Applicability

The present invention provides a protein of mammalian origin comprising a kinase activity, and the gene thereof. Since the protein of the invention is thought to be involved in the regulation of the cell cycle, the present invention enables the development of novel pharmaceutical drugs for the diagnosis and treatment of proliferative disorders such as cancer. Also, the gene encoding the protein of the invention is expected to be utilized in the gene therapy of the above-mentioned diseases. The present invention also features a host-vector system for producing the protein of the invention, and thereby, the mass production of the protein of the invention became possible. Furthermore, an oligonucleotide that specifically hybridizes to the DNA encoding the protein of the invention, and an antibody that binds to the protein of the invention are also provided by the present invention, enabling easy detection and isolation of the protein of the invention and its gene. Furthermore, the present invention also provides a compound that binds to the protein of the invention and a method of screening a compound that promotes or inhibits the activity of the protein. The compound thus isolated is useful as a drug candidate compound for diagnosing and treating the above-mentioned diseases. Especially, a compound that inhibits the activity and expression of the protein of the invention is anticipated to be used as an anti-cancer agent.

## Claims

1. A protein comprising the amino acid sequence of SEQ ID NO: 1.

2. A protein comprising the amino acid sequence of SEQ ID NO: 1, in which one or more amino acids are replaced, deleted, inserted, and/or added, and having a kinase activity.

3. A protein encoded by a mammalian-derived DNA that hybridizes to the DNA comprising the nucleotide sequence of SEQ ID NO: 2, said protein having a kinase activity.

4. A DNA encoding the protein of any one of claims 1 to 3.

5. A vector comprising the DNA of claim 4.

6. A transformant carrying the DNA of claim 4 in an expressible manner.

7. A method for producing the protein of any one of claims 1 to 3, the method comrising a step of culturing the transformant of claim 6.

8. An antibody that binds to the protein of any one of claims 1 to 3.

9. A DNA specifically hybridizing to the DNA of claim 4, and comprising at least 15 nucleotides.

10. A method for screening a compound having the activity to bind to the protein of any one of claims 1 to 3, the method comprising the steps of,
(a) contacting a test sample with the protein of any one of claims 1 to 3, and,
(b) selecting a compound having the activity to bind to the protein of any one of claims 1 to 3.

11. A method for screening a compound that can promote or inhibit the kinase activity of the protein of any one of claims 1 to 3, the method comprising the steps of,
(a) contacting the protein of any one of claims 1 to 3 with the corresponding substrate under the presence of a test compound,
(b) assaying the substrate-targeted kinase activity of the protein of any one of claims 1 to 3, and
(c) selecting a compound that can promote or inhibit the substrate-targeted kinase activity of the protein of any one of claims 1 to 3, by comparing the activity with that obtained in the assay under the absence of the test compound (control).

12. A compound that can be isolated by the method of claims 10 or 11.

13. The compound of claim 12, wherein the compound is a protein.

14. The compound of claim 13, wherein the protein is an antibody.

15. A pharmaceutical composition comprising a compound that inhibits the kinase activity of the protein of any one of claims 1 to 3.

16. A pharmaceutical composition comprising a compound that inhibits the intracellular expression of the DNA of claim 4.

17. The pharmaceutical composition of claims 15 or 16, wherein the pharmaceutical composition is an anti-cancer drug.
